## Europäisches Patentamt
## European Patent Office
## Office européen des brevets

(11) Publication number: **0 040 058**
**B1**

(12) **EUROPEAN PATENT SPECIFICATION**

(45) Date of publication of patent specification: **21.08.85**

(51) Int. Cl.⁴: **G 01 N 33/554**, G 01 N 33/58

(21) Application number: **81302036.9**

(22) Date of filing: **08.05.81**

(54) **Method for detection of oncofetal antigen, for detection of cancer, for evaluation for cancer therapy and diagnostic kit suitable for such purpose.**

(30) Priority: **08.05.80 US 147928**

(43) Date of publication of application:
**18.11.81 Bulletin 81/46**

(45) Publication of the grant of the patent:
**21.08.85 Bulletin 85/34**

(84) Designated Contracting States:
**AT BE CH DE FR GB IT LI NL SE**

(56) References cited:
**DE-A-2 639 623**
**US-A-3 988 115**
**US-A-4 116 776**
**US-A-4 140 753**
**US-A-4 144 031**

**CANCER IMMUNOLOGY AND IMMUNOTHERAPY, Vol. 3, 1977 C. MOROZ et al. "Ferritin-Bearing Lymphocytes and T-Cell Levels in Peripheral Blood of Patients with Breast Cancer" pages 101 to 105**
**ANNUAL REVIEW OF MEDICINE, Vol. 28, 1977 T.B. TOMASI "Structure and Function of Alpha-Fetprotein" pages 453 to 465**

(73) Proprietor: **Corning Glass Works**
**Houghton Park**
**Corning New York 14831 (US)**

(72) Inventor: **Bluestein, Barry Ira**
**R.D. 1, Wollet Road**
**Beaver Dams, New York (US)**
Inventor: **Odstrchel, Gerald**
**72 Pine Circle**
**Horseheads, New York (US)**
Inventor: **Luderer, Albert August**
**R.D. 3, P.O. Box 96A Goff Road**
**Corning New York (US)**

(74) Representative: **Smith, Sydney et al**
**Elkington and Fife High Holborn House 52/54**
**High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to diagnostic procedures and clinical test kits for determining the presence of an oncofetal antigen on the surface of mononuclear blood cells. More particularly, the present invention relates to improved immunoassay procedures of the antigen-antibody type wherein a unique positive control is employed to provide a means for checking the viability of reagents and to provide test reliability. Semi-quantitative and quantitative embodiments are disclosed.

U.S. Patent No. 4,116,776 to Dalbow et al. discloses a diagnostic procedure for determining the presence of human chorionic gonadotropin (hCG) on lymphocytes. The procedure is directed to an improvement in the detection of hCG in men and non-pregnant women as a test for cancer. Dalbow et al. note the conventional radioimmunoassay test for the presence of hCG in blood serum and then state that they have found that the host's lymphocytes collect and bind the hCG secreted by malignant neoplastic cells and, because of this, their test is characterised by a sensitivity not found in the conventional serum test. After removing sialic acid residues from the hCG molecules bound to the lymphocytes, Dalbow et al. expose the lymphocytes to labeled antiserum specific to the beta-subunit of hCG and, after equilibrium is reached, measure the concentration of hCG by measuring the amount of label, such as fluorescence or radioactivity. Negative controls, such as blood from known healthy individuals or a selected non-specific antiserum attached to the same label, are described to be normally employed in the Dalbow et al. assay.

U.S. Patent No. 3,988,115 to Modabber discloses a diagnostic procedure for determining the amount of lymphocytes capable of binding to a ligand. The test is based on a ligand interacting with lymphocytes having receptors specific to the ligand, and in this manner stimulating the proliferation of only the lymphocytes having the receptors specific to the ligand. Labeled ligand is used to detect the quantity of the lymphocytes having the receptors specific to the ligand. One advantage of the test is stated to be its usefulness prior to the presence of a significant amount of antibody in the blood stream. Blood from a normal individual or particles of predetermined ligand binding activity, such as antibody absorbed onto gel beads, can be used as a control.

Rockoff et al., "Sensitive and Convenient Quantitation of Antibody Binding to Cellular Antigens Using Glutaraldehyde Preserved Cells", *Journal of Immunological Methods, 26,* 369 (1979), describe a procedure to detect and quantify antibodies specific to cancers using glutaraldehyde-fixed target cells. The controls are normal serum and liquid medium.

Tomasi, Jr., "Structure and Functions of Alphafetoprotein", *Ann. Rev. Med.,* 1977, 28:453, describes a high incidence of alpha-fetoprotein (AFP, an octofetal antigen) on the surface of peripheral blood mononuclear cells of so-called cancer-prone families. The present inventors have been informed that Tomasi, Jr. used a fluorescent immunoassay technique where a monospecific fragment of anti-AFP antibody was chemically coupled to fluorescein and then incubated with patient lymphocytes followed by counting positive cells under a microscope.

Naughton et al., "Localization of the β Chain of Human Chorionic Gonadotropin on Human Tumor Cells and Placental Cells", *Cancer Research, 35,* 1887 (1975), located hCG on the surfaces of human tumor cells using enzyme-labeled hCG antibody.

Ferritin, another oncofetal protein, has been described as a potential tumor marker. Marcus et al., "Serum Ferritin Levels in Patients with Breast Cancer", *Clin. Res.,* 23, 447 (1975) measured serum ferritin using a double-antibody radioimmunoassay technique. On the other hand, more recent work by Moroz et al. indicates that a more specific malignant tumor marker is the presence of significant ferritin-bearing lymphocytes ("Lymphocytes Bearing Surface Ferritin in Patients with Hodgkin's Disease and Breast Cancer", *The New England Journal of Medicine,* correspondence *276,* No. 20, 1172 (1977) and "Ferritin-Bearing Lymphocytes and T-Cell Levels in Peripheral Blood of Patients with Breast Cancer", *Cancer Immunol. Immunother., 3* 101 (1977)). Moroz et al. used a cytotoxicity test to determine the ferritin-bearing cells wherein after antibody is added to the patient's lymphocytes, complement is added to lyse cells which have antibody on the surface and then the number of dead cells is determined using a standard dye of the type which can't be excluded by dead cells.

It is an object of this invention to provide a diagnostic blood test for detecting at least one oncofetal antigen on peripheral blood lymphocytes.

Another object of this invention is to provide a diagnostic blood test for detecting ferritin on peripheral blood lymphocytes.

A further object of this invention is to provide a diagnostic procedure for quantifying the subpopulation of peripheral blood lymphocytes carrying at least one oncofetal antigen, such as ferritin.

Still another object of this invention is to provide an improved immunoassay label-type procedure for determining the presence of an oncofetal antigen on peripheral blood lymphocytes in a quantitative or semi-quantitative fashion wherein a positive control consisting of the oncofetal antigen attached to lymphocytes is used to validate the test system.

Another object of this invention is to provide an immunoassay label-type procedure useful in the early detection of and prognosis of at least certain types of cancer.

A further object of this invention is to provide diagnostic kits for carrying out the above-described types of diagnostic procedures.

In one embodiment, the present invention provides a method for detecting oncofetal antigen on peripheral blood mononuclear cells characterised in that it comprises:

(a) carrying out a first test by admixing an aliquot of a white blood cell sample comprising peripheral blood mononuclear cells with an aliquot of labeled oncofetal antibody for said antigen, removing the antibody which does not attach to said cells and determining the relative amount of antibody attached to said cells through the use of said labeled antibody;

(b) carrying out a second test by admixing an aliquot of said blood sample with an aliquot of labeled human oncofetal antibody and a competitive binding inhibiting amount of the oncofetal antigen, removing the antibody which is not attached to said cells and determining the relative amount of antibody attached to said cells through the use of said labeled antibody;

(c) determining the relative amount of said antigen carried by said cells from the values obtained in steps (a) and (b);

(d) repeating steps (a)—(c) but substituting peripheral blood mononuclear-type cells carrying a known quantity of the oncofetal antigen for the blood sample; and

(e) checking the validity of the method and of the labeled antibody reagent from the results of step (d).

Such a method may further comprise, in addition to said steps (a)—(e):

(f) preparing a standard curve by admixing increasing amounts of the antigen with a defined amount of immobilized antibody and a defined amount of labeled antibody;

(g) determining specific binding for each antigen concentration through the use of said label; and

(h) comparing the result obtained in step (c) with said standard curve to determine the amount of antigen corresponding to said value obtained in step (c).

Also, in addition to said steps (a)—(h), the following step (i) may be carried out.

(i) repeating steps (a)—(h) but substituting peripheral blood mononuclear-type cells carrying a protein substance known to produce a negative reaction with the oncofetal antibody for the oncofetal antigen-carrying peripheral blood mononuclear-type cells.

Preferably, the peripheral blood mononuclear cells or lymphocytes are separated from said white blood cell sample and used to carry out the above method.

In another embodiment, the present invention provides a method for detecting the presence of cancer in a patient and/or evaluating the effectiveness of cancer therapy which comprises carrying out such a method and then comparing the value obtained in step (c) or step (h) with the range of values obtained with healthy, non-malignant individuals.

In a further embodiment, the present invention provides a diagnostic kit useful for detecting oncofetal antigen on peripheral blood mononuclear cells, said kit comprising as separate reagents:

(1) labeled human oncofetal antibody for said oncofetal antigen;

(2) said oncofetal antigen; and

(3) peripheral blood mononuclear-type cells carrying a known quantity of said oncofetal antigen.

The antibody is preferably immobilized on a substrate. Reagent (3) is preferably antigen-carrying cultured lymphocytes. For example, the oncofetal antigen being ferritin, reagent (3) is cultured lymphocytes carrying ferritin.

Generally, the oncofetal antigen is preferably selected from alpha-fetoprotein, ferritin, carcino-embryonic antigen, human chorionic gonado-tropin, pancreatic associated antigen, and fetosulfoglycoproteins. The label is preferably radioactive, enzymatic or fluorescent.

In more general terms now, it has been found that at least one oncofetal antigen present on the surfaces of peripheral blood mononuclear cells, e.g. lymphocytes, can be detected in a semi-quantitative or quantitative manner by carrying out an immunoassay procedure wherein (A) an aliquot of a patient's peripheral mononuclear cells is admixed with (1) labeled antibody for the oncofetal antigen and a second aliquot of the same cell sample is admixed with (2) the labeled antibody plus a competitive binding inhibiting amount of the purified oncofetal human antigen and (b) duplicating the above procedure (a) with known antigen-positive peripheral blood mononuclear-type cells being substituted for the patient's test samples. Thereafter, appropriate calculations are carried out, as will be explained more fully hereinbelow.

In a preferred embodiment of the invention, where a quantitative assay is to be carried out, a fourth reagent, namely immobilized antibody to oncofetal antigen, is used in combination with the labeled antibody and the purified antigen to develop a series of standard curves.

In another preferred embodiment of the invention, the oncofetal antigen is human ferritin and, of course, the antibody is anti-human ferritin, preferably labeled with radioactive iodine such as $I^{125}$.

The present invention also provides reagent kits containing in separate fashion each of the reagents disclosed herein, with the immobilized antibody being included where standard curves for quantitative testing are to be developed.

In the past few years, an increasing amount of bio-chemical work has been carried out with the objective of detecting and quantifying oncofetal antigens. Oncofetal antigens normally are present in significant quantities only in fetal life, or in adults during certain non-malignant physiological conditions, such as the presence of hCG in normal pregnant women. However, there is increasing evidence that oncofetal antigens also are present in adults exhibiting or having a predisposition for cancer. A problem is that indicated above, namely, the presence of oncofetal antigens in adults due to other physiological conditions, e.g. hCG in the serum of nonpregnant women having inflammatory diseases of the digestive tract, alpha-fetoprotein in the serum of patients with acute hepatitis and chronic liver disease, and so on. Recent work reported in the literature has distinguished between serum oncofetal antigen and oncofetal antigen bound to mononuclear peripheral blood cells. It has been described in the literature that significant amounts of oncofetal antigen related to a malignant state, or even a predisposition thereto, are found on the surface of the mononuclear peripheral blood cells, instead of in the serum. Furthermore, the literature strongly suggests that the subpopulation of mononuclear peripheral blood cells carrying oncofetal antigen is independent of the serum level of oncofetal antigen, but is related to the presence of a predisposition to malignancy.

A number of researchers have focussed upon ferritin and its possible relationship to normal and diseased states in man. Ferritin is a normal iron storage protein with a molecular weight of about 450,000. Ferritin in actuality is composed of a plurality of sub-units, designated as iso-ferritins and being of about 18,000 molecular weight each, which are non-covalently bound to one another to constitute the ferritin molecule. Separation of at least some isoferritins from one another can be accomplished by using their isoelectric points. A particularly interesting range of isoferritins are those having isoelectric points within about 4.3 to 5.0. See Alpert et al., "Carcino-Foetal Human Liver Ferritins", *Nature, 242,* 194 (March 16, 1973).

Alpha-2-H-globulin, an acidic isoferritin found primarily in the fetal liver (classified as an oncofetal antigen), also is found in adults with various malignancies such as hepatoma and teratoma. Immunologically, the various isoferritins have not been distinguished from normal human ferritins, although it is contemplated that through immunological antibody-producing techniques, it will be possible to produce antibody for specific isoferritins, such as the above-mentioned alpha-2-H-globulin, and isoferritins having isoelectric points within the 5.1 to 5.6 range.

It is difficult to use serum ferritin levels as an accurate diagnostic tool in view of the above-described naturally occurring biological phenomena, namely, the presence of ferritins in the normal blood serum over a fairly wide concentration range, inability at this time to immunologically distinguish between different isoferritins, and, in addition, the presence of elevated serum ferritin levels in some non-malignant disease states, such as a nonmalignant anemia. In one study, about 70 percent of the patients tested and having malignancy exhibited elevated serum ferritin levels. It also has been reported that in early breast cancer serum ferritin levels overlap the normal serum ferritin concentration range, although extremely elevated levels are found in women with recurrent or metastatic carcinoma of the breast. Jacobs et al., "Serum Ferritin Concentration in Early Breast Cancer", *Br. J. Cancer, 34* (3), 286 (1976) and "Serum Ferritin Concentration in Untreated Hodgkin's Disease", *ibid, 34*(3), 162 (1976); Sarcinone et al., "Increased Ferritin Synthesis and Release by Hodgkin's Disease in Peripheral Blood Lymphocytes", *Int. J. Cancer,* 20 339 (1977) and Niitsu et al., "Radioimmunoassay of Serum Ferritin in Patients with Malignancy", *Ann. N.Y. Acad. Sci. 259,* 450 (1975).

Some of the work of Moroz et al. has been referred to hereinbefore. In addition, Moroz et al. determined that apoferritin acts as a blocking substance for T lymphocytes in Hodgkin's lymphoma, thereby reducing the tendency of such cells to spontaneously form rosettes with sheep red blood cells (Moroz et al. "Ferritin on the Surface of Lymphocytes in Hodgkin's Disease Patients—a Possible Blocking Substance Removed by Levamisole", *Clin. Exp. Immunol., 29,* 30 (1977)). This may have led to their work investigating the quantifying of ferritin-bearing lymphocytes as indicative of breast cancer, using the cytotoxicity test described hereinbefore. One of the more pronounced drawbacks of the cytotoxicity test is the need to separately evaluate the effects of each reagent used on the patient's cells.

The present invention provides a rapid, non-invasive method to detect and quantify ferritin-bearing lymphocytes. In addition to experimental laboratory usage for oncofetal antigen detection *per se*, the testing procedures of this invention have been shown to be of value in detecting and evaluating treatment of breast and other cancers.

Descriptions of the three (semi-quantitative test) or four (quantitative test) reagents required in the present invention will now be set forth.

(1) Labeled anti-human ferritin antibody

The ferritin antibody used in the studies described hereinbelow was monospecific human ferritin antibody. It is contemplated that an antibody of even greater specificity, that is, an isoferritin-specific antibody, could be prepared by substituting at least one isoferritin species or an isoelectric-determined range thereof for normal human ferritin in the procedure described below to prepare the labeled human ferritin antibody.

Rabbits are immunized with human ferritin using standard immunological procedures. The following affinity purification procedure then can be carried out to yield the monospecific antibody. Equivalent procedures will be apparent to the skilled artisan.

Arylamine controlled-pore (1350 Å) glass, 200 to 400 mesh particle size, is activated with glutaraldehyde (5 ml/gm glass). After washing to remove any non-reacted glutaraldehyde, 1 mg human ferritin per gram of glass is gently admixed with the activated glass for about 2 hours at room temperature. Thereafter, the glass is successively washed with 1 M sodium chloride, 0.1 M glycine, and then 3 to 5 washes with 0.1 M phosphate buffer. The arylamine glass is readily prepared by known procedures; see, for example, Weetall, *Science, 166*, 615 (1969), and references cited therein.

The ferritin-coupled glass is then gently admixed with the collected ferritin antiserum, about 1 ml antiserum per gram of ferritin-conjugated glass. After incubation at 22°C overnight, the glass is washed successively with 0.1 M sodium carbonate, 0.15 M sodium chloride, pH 8.0; 0.1 M sodium acetate; 0.15 M sodium chloride, pH 5.0; and two washes of distilled water adjusted to pH 4.4 with 1 M hydrochloric acid. The monospecific antibody is eluted with distilled water, adjusted to pH 2.2. The slurry is filtered into 0.3 M sodium phosphate, pH 7.35, and the filtrate concentrated by membrane ultrafiltration. Antibody can be stored in a solution of 0.1 M sodium phosphate with 0.2 percent azide.

The antibody can be coupled to any of the conventional labels, such as radioactive element, enzyme, or fluorescing agent. In the studies described herein, the monospecific human ferritin antibody was radiolabeled with $NaI^{125}$.

(2) Ferritin positive mononuclear cells

This reagent is used as a positive quality control to monitor the stability of reagent 1, above, and to validate the assay procedure.

The mononuclear cells used in preparing this reagent are usually culture-grown lymphocytes, so as to avoid fluctuations of ferritin level which can occur with naturally occurring human lymphocytes. The work described herein used acute lymphoblastic leukemia cells (CCL-119, ATCC); however, other cultured lymphocytes can be substituted therefor, such as murine lymphocytes, CCL-86 or CCL-126 lines, or even normal subject peripheral blood lymphocytes if adequate screening is employed. Such mononuclear cells, then, are peripheral blood mononuclear-type cells. Such cells need not be naturally-occurring peripheral blood mononuclear cells as long as such cells exhibit membrane surface characteristics which are similar to such naturally-occurring peripheral blood mononuclear cells.

In these studies, the positive quality control reagent cells were prepared using acute lymphoblastic leukemia cells (CCI-119) grown in RPMI-1640 medium (Grand Island Biological Co., Grand Island, N.Y.) supplemented with 10 percent fetal calf serum (FCS, Grand Island Biological Co.). These cells can be grown in continuous culture using spinner techniques and are suitable for large-scale preparation. The CCI-119 lymphocytes were harvested by centrifugation at 200 to 300 xg for 15 minutes and washed two times in phosphate-buffered saline (PBS, 0.01 M phosphate, 0.15 M sodium chloride, pH 7.4). The cells were then reconstituted to a concentration of 2 to $3\times10^7$ cells/ml in PBS. Cell concentration was measured by counting in a hemocytometer. Sufficient glutaraldehyde solution of 25 percent (w/w) was added to make the final glutaraldehyde concentration approximately 1 percent. Activation at cell densities above this concentration resulted in extensive cell aggregation. Cells were incubated with continuous rocking for 30 minutes at 22°C followed by harvesting and washing by centrifugation. Purified human ferritin obtained from JBL Chemical Company, San Lous Obispo, California was diluted with PBS to a final concentration of 200 to 300 µg/ml and added to the cell pellet in an amount sufficient to give a final cell concentration of 2 to $3\times10^7$ cells/ml.

The cells were incubated with rocking for 30 minutes at 22°C and then at 4°C for further incubation for an additional 16 to 18 hours. The cells were then washed as described above and suspended in PBS containing 1 percent bovine serum albumin (BSA) and 0.2 percent sodium azide. The average recovery of derivatized cells from the starting population was 40 to 50 percent. These cells were stored at 4°C and showed only a 10% decrease in activity after 40 days when estimated by specific binding of $I^{125}$-labeled human ferritin antibody.

Evaluation of these cells by indirect immunofluorescence using rabbit anti-human ferritin sera and fluorescein-conjugated goat anti-rabbit sera showed 43 percent fluorescent-positive cells. In contrast, CCI-119's which were not conjugated to ferritin or CCI-119's conjugated to bovine serum albumin showed no fluorescence. These positive quality control cells did not fluoresce when incubated with normal rabbit serum. The approximate loading of ferritin on these cells was determined to be 25 to 40 ng per $2.5\times10^5$ cells.

(3) Purified human ferritin

This reagent is commercially available and is used in both the semi-quantitative and quantitative test modes. In the semi-quantitative mode it is used in high concentration to competitively block labeled ferritin antibody binding to ferritin present on the patient's lymphocytes in order to assess specific labeled antibody binding. In the quantitative mode it is also used in various concentrations in conjunction with the immuno-radiometric assay (IRMA) in order to develop a standard curve from which absolute values of lymphocyte-bound ferritin may be determined.

(4) Immobilized human ferritin antiserum

This reagent is used in conjunction with reagents 1 and 3 to generate a standard curve from which values for lymphocyte-bound ferritin may be extrapolated and consists of the human ferritin anti-sera covalently coupled to a substrate such as controlled-pore glass.

In the tests described herein, this reagent was prepared using 1 µ particle size, 550 Å arylamine controlled-pore glass.

This glass was first activated at 0°C with 2 N hydrochloric acid and sodium nitrite. After 20 minutes, the glass was washed extensively with distilled water. One ml of whole human ferritin anti-serum per gram of glass then was added. The pH was adjusted to 8.4 and the reaction allowed to proceed overnight at 4°C. The immobilized antibody was washed extensively in 0.1 M phosphate buffer, pH 7.0, and stored at 4°C at a final concentration of 20 mg glass/ml in 0.01 M phosphate buffer, pH 7.0, containing 0.1 percent BSA.

Detailed descriptions of test modes using the above-described reagents to carry out the assays of this invention will be disclosed hereinbelow. In general, the first three of the reagents listed above (labeled human ferritin antibody, ferritin positive lymphocytes, and purified human ferritin) are used to perform a semi-quantitative assay while all four reagents (including the immobilized human ferritin antiserum) are used for a quantitative determination of lymphocyte-bound ferritin.

Regardless of whether quantitative or semi-quantitative tests are desired, the test component will be a sample of the patient's peripheral mononuclear blood cells, particularly the lymphocytes. Although it is believed that with careful analytical techniques, a sample of all of the patient's white blood cells can be used (such as a buffy coat), it is recommended at this time that the mononuclear peripheral blood cells be separated from other blood components as a first step. Techniques known to the skilled artisan are employed to separate the mononuclear peripheral blood cells from other blood components, such as a mononuclear cell separator (Luderer et al., "Rapid, Quantitative Human Lymphocyte Separation and Purification in a Closed System", *Molecular Immunol.*, *16*, 621 (1979)) or the Ficoll-Hypaque technique of Boyum (*Scand. J. Immunol.*, *5* (suppl. 5) 9 (1976)). Conventional washing such as by centrifugation and adjustment to a standardized cell number, such as by counting in a hemocytometer or automatic blood cell counter, will complete the preparatory phase of the assay. Although not necessary, the lymphocyte fraction of the mononuclear peripheral blood cells can be obtained by a known procedure and used as the test sample.

In the semi-quantitative mode, an aliquot of the lymphocytes is admixed with an aliquot of the labeled anti-human ferritin antibody, and, preferably, an aliquot of buffer. Separately, the patient's lymphocytes, the labeled antihuman ferritin antibody, and an aliquot of purified human ferritin, the latter also taking the place of the buffer where used, are admixed to provide the usual competitive test binding control, since the human ferritin is added in high concentration to competitively block any specific binding of the labeled antibody to the test cells. The second part of the test (which of course can be carried out before, during, or after the testing described above) involves duplicating the two separate steps above, but with the substitution of the ferritin positive lymphocytes for the test cells to monitor the functionality of the assay system and the stability of the labeled antibody. As an optional step, if desired as a further check, the second part of the test can be duplicated, but with the substitution of a control negative cell for the ferritin positive lymphocytes. The control negative cell is one carrying a protein which will not react with the labeled antibody, such as CCL-119 lymphocytes carrying bovine serum albumin.

After a suitable incubation period, the cells are washed with PBS and centrifuged. The supernatant is recovered and the pelleted cells assessed for bound label. If $I^{125}$-labeled antibody is used, pellets are counted in a gamma spectrometer. Semi-quantitative results reported as specific binding are determined by subtracting counts per minute (cpm) bound to cells in the presence of excess soluble human ferritin from cpm bound in the absence of added excess human ferritin, divided by the cpm of the total $I^{125}$-labeled antibody added to the assay system minus cpm bound to cells in the presence of added excess human ferritin. Added excess human ferritin is defined as the amount of soluble ferritin added to the assay system that will completely block specific labeled antibody binding to the cell.

Generally, specific binding is evaluated with at least two different cell concentrations. Results are reported as specific cpm or percent bound to peripheral blood lymphocytes per specified number of cells.

In the quantitative assay, patient and quality control lymphocytes are assayed as above, but a standard curve also is prepared by incubating increasing amounts of human ferritin with a fixed concentration of immobilized antihuman ferritin antiserum (IMA). In this system, IMA is first mixed with buffer alone or varying concentrations of human ferritin. At a fixed time interval, labeled antibody is added and the incubation allowed to proceed to a final time interval equal to that used in analyzing the test cells. The IMA is washed with buffer and pelleted by centrifugation. The supernatants are decanted and the pellets assessed for radioactivity. Specific binding is determined for each human ferritin concentration in a manner similar to that described above except that cpm bound to IMA in the presence of buffer alone is subtracted from both the total cpm added and cpm bound with increasing amounts of added human ferritin. A standard curve is prepared by semi-logarithmic plotting of specific binding (linear scale) versus the amount of ferritin added (log scale). By extrapolating the specific binding by patient cells to the log scale, a quantitative measure of ferritin bound to patient's peripheral blood lymphocytes is made. At the present time, it is recommended that the standard curve be developed at about the time of patient testing, using the same human ferritin to be employed in the patient testing. As standardization and shelf life, i.e., stability, are improved, standard curve generation may be carried out at infrequent intervals, realizing of course that where isotopes are employed, the standard curve should always be freshly generated.

Of course, in each of the above modes, the identical calculations are run using the results obtained in the second part of the assay with the ferritin positive lymphocytes to validate the reagents and test results.

As will be illustrated more fully hereinbelow, it is believed that the testing procedure described herein can be used as an effective tool in the early diagnosis of cancer and also to monitor the effectiveness of treatment. Normal healthy individuals will exhibit essentially no difference, or a small difference, in bound lable measured in the presence and absence of the purified human ferritin, while the peripheral blood lymphocytes from patients with early (preclinical) cancer or advanced cancer show increased bound label in the system in which the human ferritin has been omitted.

A more detailed description of an assay procedure using the reagents specifically described hereinbefore is presented below, as exemplifying the assay techniques of this invention. Variations in operating procedure will be obvious to the skilled artisan.

Anti-coagulated blood specimens were subjected to the technique of Boyum mentioned earlier for isolation of lymphocytes. This involved a 1:2 dilution of the blood with physiologic phosphate buffered saline, followed by gently layering the blood onto a solution of Ficoll-Hypaque. Following centrifugation at 400 xg for 30 minutes, the lymphocytes banded at a defined interface and were removed with a Pasteur pipette, washed two times in PBS by gentle centrifugation (200 xg, 10 minutes), and suspended in PBS-0.1% BSA 0.02% sodium azide or RPMI culture medium at a concentration of $1 \times 10^7$ cells/ml. The control positive cells (lymphocytes bearing ferritin) were also suspended at this concentration.

Four 12×75 mm plastic test tubes were allocated for each sample tested at each cell concentration. Reactions were set up in a 4°C water bath. 100 µl of cells was added to each test tube. The first two tubes of each series contained an additional 100 µl of the PBS-BSA-azide solution while the second two tubes contained 100 µl of the human ferritin in PBS-BSA-azide added at a concentration of 10 to 50 µg/ml (excess HF). These concentrations were sufficient to completely block the specific binding of $I^{125}$-labeled antibody to positive control cells at a dose of $3 \times 10^6$ cells. After addition of 100 µl $I^{125}$-labeled antibody (50,000 to 100,000 cpm), the tubes were transferred to a shaker bath and incubated overnight at 4°C (16 to 18 hours) to allow equilibrium binding to be reached. After incubation, the cells were washed with 3 ml of PBS and centrifuged at 3,000 rpm for 15 minutes. Supernatants were aspirated and cell pellets counted for bound radioactivity. Duplicates were averaged and values for specific binding calculated as described above.

The nearly complete blocking of binding by the human ferritin establishes the specificity of the labeled ferritin antibody for the ferretin bound to the test cells and the low level of non-specific adsorption to cell surfaces.

The extremely good sensitivity of the assay of this invention is discussed below.

The lower limit of sensitivity for detection of ferritin on lymphocytes was determined by competitive binding using increasing concentrations of human ferritin, a fixed concentration of radiolabeled ferritin antibody and $2.5 \times 10^5$ positive control cells (CCl-119 bearing a defined amount of ferritin). A lower limit of detection of 2 to $2.5 \times 10^{-9}$ g or 4.4 to $5.5 \times 10^{-6}$ nanomoles was found. This limit was based on the minimal amount of human ferritin which would cause a reproducible inhibition of specific binding of radiolabeled ferritin antibody to positive control cells.

The minimum detectable number of ferritin bearing cells in a population of negative cells was studied using control positive cells mixed with negative cells at a final cell population of $2.5 \times 10^5$ cells. At this cell density, 12,500 cells could be detected at a discriminating level of 5 percent specific binding. From quantative data described above, this corresponded to an absolute ferritin value of approximately 2 ng, the lower limit of detection. Therefore, in a population of $1 \times 10^6$ cells, 1.25 percent positive cells would be detectable. This is significant since the literature has suggested that 16.6 percent ferritin positive cells of the total lymphocyte population is indicative of breast cancer. (Moroz et al., *Cancer Immunol. Immunother.*, 3, 101 (1977)).

In the quantitative assay, a typical method to develop the standard curve is as follows:

To duplicate 12×75 mm plastic tubes there is added 100 µl of the human ferritin antiserum IMA previously diluted 1:10 with PBS-BSA-azide solution from a stock IMA of 20 mg glass per ml. To each set of duplicates there is added, in 100 µl, buffer alone or human ferritin diluted to various amounts with buffer. This assay is set up in a 4°C water bath. The tubes are then incubated in a shaker bath at 22°C for 30 minutes. At the end of this time, radiolabeled ferritin antibody (100 µl) is added to each tube containing IMA and incubation allowed to proceed for an additional 30 minutes. The tubes are washed and centrifuged in a fashion identical to patient or positive reference control samples. Unlike patient or reference samples, the radiolabeled antibody is not added at the time when the assay is set up, but 30 minutes later. This is to allow human ferritin to reach equilibrium with the IMA antiserum, prior to addition of labeled antiserum. However, the labeled antiserum can be added at the same time, with an appropriate incubation period.

When specific binding for positive control cells diluted to various concentrations is extrapolated from the standard curve, the absolute amount of ferritin bound can be determined.

Table 1 sets forth testing results with a number of patients using the assay of this invention in a semi-quantitative mode. The percent accuracy was developed from other diagnostic procedures, such as biopsy, which also determined the patient's category.

TABLE 1

| Category | Number patients | Number* positive patients | Number* negative patients | % Accuracy |
|---|---|---|---|---|
| Metastatic disease (active) | 23 | 12 | 11 | 52 |
| Metastatic disease (remission) | 25 | 0 | 25 | 100 |
| Pre-operative (primary) | 16 | 4 | 12 | 25 |
| Benign breast | 36 | 1 | 35 | 97 |
| Hodgkin's disease | 3 | 2 | 1 | 67 |

*based on the lymphocyte-ferritin assay procedure.

In evaluating the results tabulated in Table 1, it is apparent that the test of this invention may have a unique value in screening out non-malignant patients and in indicating the effectiveness of treatment.

Table 2 sets forth actual mean values of ferritin positive lymphocytes using the semi-quantitative procedure at 4°C with 16—18 hour incubation using radiolabeled ($I^{125}$) anti-human ferritin antibody. These values, although based on a limited number of patients, show the relative values between malignant and non-malignant states.

TABLE 2

1. Normal women bind a mean of 2 percent with a range of 0.4 to 4.5 percent, the higher values being found in women over 50 years of age.

2. Benign breast diseases including galactorrhea, fibrocystic disease and fibroadenoma bind a mean of 1.6 percent, a value well within the normal range.

3. Women who have undergone mastectomy and show no evidence of recurrent disease or are responsive to radiation and chemotherapy have reduced binding values (mean 2.5 percent) relative to those women with metastatic disease who do not respond to therapy (mean 7 percent).

4. Women with breast cancer, prior to undergoing surgery, show elevated binding levels (6.5 to 10 percent).

Variations of the invention will be apparent to the skilled person. For example, various coupling agents, such as carbodiimide or diisothiocyanates could be used in place of the glutaraldehyde; other immobilization substrates such as starch or gel beads could be employed in place of the glass; other conventional standard curve development procedures could be employed; other oncofetal antigens, such as alphafetoprotein, carcinoembryonic antigen, human chorionic gonadotropin, pancreatic associated antigen, fetosulfoglyco-proteins etc., with development of the appropriate antibody, could be used in place of ferritin, and so forth.

Claims

1. A method for detecting oncofetal antigen on peripheral blood mononuclear cells characterised in that it comprises:

(a) carrying out a first test by admixing an aliquot of a white blood cell sample comprising peripheral blood mononuclear cells with an aliquot of labeled oncofetal antibody for said antigen, removing the antibody which does not attach to said cells and determining the relative amount of antibody attached to said cells through the use of said labeled antibody;

(b) carrying out a second test by admixing an aliquot of said blood sample with an aliquot of labeled human oncofetal antibody and a competitive binding inhibiting amount of the oncofetal antigen, removing the antibody which is not attached to said cells and determining the relative amount of antibody attached to said cells through the use of said labeled antibody;

(c) determining the relative amount of said antigen carried by said cells from the values obtained in steps (a) and (b);

(d) repeating steps (a)—(c) but substituting peripheral blood mononuclear-type cells carrying a known quantity of the oncofetal antigen for the blood sample; and

(e) checking the validity of the method and of the labeled antibody reagent from the results of step (d).

2. A method as claimed in claim 1 characterised in that a quantitative detection method is carried out which comprises, in addition to said steps (a)—(e):

(f) preparing a standard curve by admixing increasing amounts of the antigen with a defined amount of immobilized antibody and a defined amount of labeled antibody;

(g) determining specific binding for each antigen concentration through the use of said label; and

(h) comparing the result obtained in step (c) with said standard curve to determine the amount of antigen corresponding to said value obtained in step (c).

3. A method as claimed in claim 1 or claim 2 characterised in that the peripheral blood mononuclear cells are separated from said white blood cell sample and used to carry out the method.

4. A method as claimed in claim 1 or claim 2 characterised in that the peripheral blood lymphocytes are separated from said white blood cell sample and used to carry out the method.

5. A method as claimed in any of claims 1 to 3 characterised in that the oncofetal antigen is selected from alpha-fetoprotein, ferritin, carcinoembryonic antigen, human chorionic gonadotropin, pancreatic associated antigen, and fetosulfoglycoproteins.

6. A method as claimed in any of claims 1 to 3 and 5 characterised in that the label is radioactive, enzymatic, or fluorescent.

7. A method for detecting the presence of cancer in a patient and/or evaluating the effectiveness of cancer therapy which comprises carrying out a method as claimed in claim 1 and then comparing the value obtained in step (c) with the range of values obtained with healthy, non-malignant individuals.

8. A method for detecting the presence of cancer in a patient and/or evaluating the effectiveness of cancer therapy which comprises carrying out a method as claimed in claim 2 and then comparing the value obtained in step (h) with the range of values obtained with healthy, non-malignant individuals.

9. A method as claimed in claim 7 or claim 8 characterised in that the oncofetal antigen is selected from alpha-fetoprotein, ferritin, carcino-embryonic antigen, human chorionic gonado-tropin, pancreatic associated antigen, and fetosulfoglycoprotein.

10. A diagnostic kit useful for detecting oncofetal antigen on peripheral blood mononuclear cells, said kit comprising as separate reagents:

(1) labeled human oncofetal antibody for said oncofetal antigen;

(2) said oncofetal antigen; and

(3) peripheral blood mononuclear-type cells carrying a known quantity of said oncofetal antigen.

11. A diagnostic kit as claimed in claim 10 characterised in that it includes the antibody immobilized on a substrate.

12. A kit as claimed in claim 10 or claim 11 characterised in that the label is radioactive, enzymatic, or fluorescent.

13. A kit as claimed in any of claims 10 to 12 characterised in that the oncofetal antigen is selected from alpha-fetoprotein, ferritin, carcino-embryonic antigen, human chorionic gonado-tropin, pancreatic associated antigen, and fetosulfoglycoprotein.

14. A kit as claimed in any of claims 10 to 13 characterised in that reagent (3) is cultured lymphocytes carrying said antigen.

15. A kit as claimed in any of claims 10 to 13 characterised in that the oncofetal antigen is ferritin and said reagent (3) is cultured lymphocytes carrying ferritin.

16. A method as claimed in claim 2 characterised in that in addition to said steps (a)—(h) the following step (i) is carried out:

(i) repeating steps (a)—(h) but substituting peripheral blood mononuclear-type cells carrying a protein substance known to produce a negative reaction with the oncofetal antibody for the oncofetal antigen-carrying peripheral blood mononuclear-type cells.

## Patentansprüche

1. Verfahren zur Ermittlung von oncofötalem Antigen an peripherischen mononuklearen Blutzellen, gekennzeichnet durch:

a) die Durchführung eines ersten Tests, in dem ein Aliquot einer weißen Blutzellenprobe, die mononukleare peripherische Blutzellen umfaßt, mit einem Aliquot eines markierten oncofötalen Antikörpers für dieses Antigen vermischt wird, der Antikörper, der nicht an den besagten Zellen haftet, entfernt wird und die relative Menge des Antikörpers, der an den besagten Zellen haftet, unter Verwendung eines markierten Antikörpers bestimmt wird;

b) die Durchführung eines zweiten Tests, in dem ein Aliquot der besagten Blutprobe mit einem Aliquot eines markierten menschlichen Oncofötalantikörpers und einer komeptitiven bindungshemmenden Menge des oncofötalen Antikörpers vermischt werden, der Antikörper, der nicht an die besagten Zellen gebunden ist, entfernt wird und die relative Menge des Antikörpers, der an die besagten Zellen gebunden ist, unter Verwendung des besagten markierten Antikörpers bestimmt wird;

c) die Bestimmung der relativen Menge des besagten Antigens, das von den besagten Zellen getragen wird, aufgrund der Werte, die bei den Stufen (a) und (b) erhalten werden;

d) die Wiederholung der Stufen a)—c), jedoch unter Ersatz der Blutprobe durch peripherische Blutzellen vom mononuklearen Typ, welche eine bekannte Menge des oncofötalen Antigens tragen; und

e) die Überprüfung der Genauigkeit des Verfahrens und des markierten Antikörper-Reagenz aufgrund der Ergebnisse der Stufe (d).

2. Verfahren nach Anspruch 1, dadurch gekenn-zeichnet, daß ein quantitatives Bestimmungs-verfahren durchgeführt wird, das zusätzlich zu den besagten Schritten (a) bis 8e) folgenden Schritte umfaßt:

(f) die Erstellung einer Standardkurve durch Vermischen zunehmender Mengen des Antigens mit einer definierten Menge immobilisiertem Antikörper und einer definierten Menge markiertem Antikörper;

(g) Bestimmung der spezifischen Bindung bei jeder Antigenkonzentration unter Verwendung der besagten Markierung; und

(h) den Vergleich der Ergebnisse, die bei der Stufe c) erhalten werden, mit der Standardkurve, um die Menge des Antigens zu bestimmen, das dem Wert entspricht, der in der Stufe c) erhalten wird.

3. Verfahren nach Anspruch 1 oder 2, dadurch gekennzeichnet, daß die peripherischen mononuklearen Blutzellen von der besagten weißen Blutzellenprobe abgetrennt und zur Durchführung des Verfahrens eingesetzt werden.

4. Verfahren nach Anspruch 1 oder 2, dadurch gekennzeichnet, daß die peripherischen Blutlymphocyten von der weißen Blutzellenprobe abgetrennt und zur Durchführung des Verfahrens eingesetzt werden.

5. Verfahren nach einem der Ansprüche 1 bis 3, dadurch gekennzeichnet, daß das oncofötale Antigen ausgewählt wird aus alpha-Fötoprotein, Ferritin, carcinoembryonischem Antigen, chorionischem Humangonadotropin, Pankreas-assoziiertem Antigen und Fötosulfoglycoproteinen.

6. Verfahren nach einem der Ansprüche 1 bis 3, sowie 5, dadurch gekennzeichnet, daß die Markierung radioaktiv, enzymatisch oder fluoreszierend ist.

7. Verfahren zur Bestimmung der Anwesenheit von Krebs bei einem Patienten und/oder Bewertung der Effektivität eine Krebstherapie, bei dem ein Verfahren nach dem Anspruch 1, durchgeführt und dann die beim Schritt (c) erhalten Werte mit dem Bereich der Werte verglichen werden, die bei gesunden, nichtmalignen Personen erhalten werden.

8. Verfahren zur Bestimmung der Anwesenheit von Krebs in einem Patienten und/oder Bewertung der Effektivität einer Krebstherapie, bei dem ein Verfahren nach dem Anspruch 2 durchgeführt wird und dann die beim Schritt (h) erhaltenen Werte mit dem Bereich der Werte verglichen werden, die mit gesunden, nichtmalignen Personen erhalten werden.

9. Verfahren nach Anspruch 7 oder 8, dadurch gekennzeichnet, daß das oncofötale Antigen ausgewählt wird aus alpha-Fötoprotein, Ferritin, carcinoembryonischem Antigen, chorionischem Humangonadotropin, Pankreasassoziiertem Antigen und Fötosulfoglycoproteinen.

10. Diagnostisches Besteck (Kit) zur Bestimmung von oncofötalem Antigen an peripherischen mononuklearen Blutzellen, welches Besteck als separate Reagenzien umfaßt:

(1) markierten humanoncofötalen Antikörper für das besagte oncofötale Antigen;

(2) besagtes oncofötales Antigen; und

(3) peripherische Blutzellen vom mononuklearen Typ, die eine bekannte Menge des oncofötalen Antigens tragen.

11. Besteck nach Anspruch 10, dadurch gekennzeichnet, daß es den Antikörper auf einem Substrat immobilisiert aufweist.

12. Besteck nach Anspruch 10 oder 11, dadurch gekennzeichnet, daß die Markierung radioaktiv, enzymatisch oder fluoreszierend ist.

13. Besteck nach einem der Ansprüche 10 bis 12, dadurch gekennzeichnet, daß das oncofötale Antigen ausgewählt wird aus alpha-Fötoprotein, Ferritin, carcinoembryonischem Antigen, chorionischem Humangonadotropin, Pankreas-assoziiertem Antigen und Fötosulfoglycoproteinen.

14. Besteck nach einem der Ansprüche 10 bis 13, dadurch gekennzeichnet, daß das Reagenz (3) aus kultivierten Lymphocyten besteht, die besagtes Antigen tragen.

15. Besteck nach einem der Ansprüche 10 bis 13, dadurch gekennzeichnet, daß das oncofötale Antigen Ferritin ist und das Reagenz (3) aus kultivierten Lymphocyten besteht, die Ferritin tragen.

16. Verfahren nach Anspruch 2, dadurch gekennzeichnet, daß zusätzlich zu den Schritten (a)—(h) der folgende Schritt (i) durchgeführt wird:

(i) die Schritte (a)—(h) werden wiederholt, jedoch werden die das oncofötale Antigen tragenden peripherischen Blutzellen vom mononuklearen Typ durch peripherische Blutzellen vom mononuklearen Typ, die eine Proteinsubstanz tragen, die mit dem oncofötalem Antikörper bekanntermaßen eine negative Reaktion erzeugen, ersetzt.

**Revendications**

1. Méthode pour la détection d'antigène oncofoetal sur des cellules sanguines mononucléaires périphériques caractérisée en ce qu'elle comprend les opérations consistant:

(a) à effectuer un premier test en mélangeant une partie aliquote d'un échantillon de globules blancs comprenant des globules sanguins mononucléaires périphériques avec une partie aliquote d'anticorps oncofoetal marqué pour cet antigène, à éliminer l'anticorps qui ne se fixe pas auxdits globules et à déterminer la quantité relative d'anticorps fixé auxdits globules par utilisation dudit anticorps marqué;

(b) à effectuer un second test en mélangeant une partie aliquote dudit échantillon de sang avec une partie aliquote d'anticorps oncofoetal humain marqué et avec une quantité inhibitrice de fixation compétitive de l'antigène oncofoetal, à éliminer l'anticorps qui n'est pas fixé auxdits globules et à déterminer la quantité relative d'anticorps fixé auxdits globules par utilisation dudit anticorps marqué;

(c) à déterminer la quantité relative dudit antigène porté par lesdits globules d'après les valeurs obtenues dans les étapes (a) et (b);

(d) à répéter les opérations (a) à (c), mais en substituant des cellules sanguines mononucléaires périphériques portant une quantité connue de l'antigène oncofoetal pour l'échantillon sanguin; et

(e) à contrôler la validité de la méthode et du réactif anticorps marqué d'après les résultats de l'étape (d).

2. Méthode selon la revendication 1, caractérisée par la mise en oeuvre d'une méthode de détection quantitative qui comprend, en plus des opérations (a), à (e) précitées:

(f) la préparation d'une courbe étalon par mélange de quantités croissantes de l'antigène avec une quantité définie d'anticorps immobilisé et une quantité définie d'anticorps marqué;

(g) la détermination de la fixation spécifique pour chaque concentration d'antigène par utilisation dudit marqueur; et

(h) la comparaison du résultat obtenu à l'étape (c) avec ladite courbe d'étalonnage à l'effet de déterminer la quantité d'antigène correspond à la valeur obtenue dans l'étape (c).

3. Méthode selon la revendication 1 ou la revendication 2, caractérisée en ce que les globules sanguins mononucléaires périphériques sont séparés dudit échantillon de globules blancs et utilisés pour mettre en oeuvre la méthode.

4. Méthode selon la revendication 1 ou la revendication 2, caractérisée en ce que les lymphocytes sanguins périphériques sont séparés dudit échantillon de globules blancs et utilisés pour mettre en oeuvre la méthode.

5. Méthode selon l'une quelconque des revendications 1 à 3, caractérisée en ce que l'antigène oncofoetal est choisi parmi l'alpha-foetoprotéine, la ferritine, l'antigène carcinomebryonnaire, la gonadotrophine chorionique humaine, l'antigène associé pancréatique et les foetosulfoglycoprotéines.

6. Méthode selon l'une quelcone des revendications 1 à 3 et 5, caractérisée en ce que le marqueur est radioactif, enzymatique ou fluorescent.

7. Méthode pour la détection de la présence d'un cancer chez un patient et/ou pour l'évaluation de l'efficacité d'une thérapeutique contre le cancer, qui comprend la mise en oeuvre d'une méthode selon la revendication 1, puis la comparaison de la valeur obtenue à l'étape (c) avec la gamme de valeurs obtenues chez les individus non atteints du cancer et bien portants.

8. Méthode pour la détection de la présence d'un cancer chez un patient et/ou pour l'évaluation de l'efficacité d'une thérapeutique contre le cancer qui comprend la mise en oeuvre d'une méthode selon la revendication 2, puis la comparaison de la valeur obtenue à l'étape (h) avec la gamme de valeurs obtenues chez les sujets non atteints du cancer, et bien portants.

9. Méthode selon la revendication 7 ou la revendication 8, caractérisé en ce que l'antigène oncofoetal est choisi parmi l'alpha-foetoprotéine, la ferritine, l'antigène carcinomebryonnaire, la gonadotrophine chorionique humaine, l'antigène associé pancréatique et la foetosulfoglycoprotéine.

10. Nécessaire de diagnostic utile pour la détection d'antigène oncofoetal sur des globules sanguins mononucléaires périphériques, ce nécessaire comprenant sous forme de réactifs séparés;

(1) de l'anticorps oncofoetal humain marqué pour ledit antigène oncofoetal;

(2) l'antigène oncofoetal; et

(3) des cellules sanguines mononucléaires périphériques portant une quantité connue dudit antigène oncofoetal.

11. Nécessaire de diagnostic selon la revendication 10, caractérisé en ce qu'il comporte l'anticorps immobilisé sur un substrat.

12. Nécessaire selon la revendication 10 ou la revendication 11, caractérisé en ce que le marquer est radioactif, enzymatique ou fluorescent.

13. Nécessaire selon l'une quelconque des revendications 10 à 12, caractérisé en ce que l'antigène oncofoetal est choisi parmi l'alpha-foetoprotéine, la ferritine, l'antigène carcinomebryonnaire, la gonadotrophine chorionique humaine, l'antigène associé pancréatique, et la foetosulfoglycoprotéine.

14. Nécessaire selon l'une quelconque des revendications 10 à 13, caractérisé en ce que le réactif (3) est formé par des lymphocytes de culture portant ledit antigène.

15. Nécessaire selon l'une quelconque des revendications 10 à 13, caractérisé en ce que l'antigène oncofoetal est de la ferritine et en ce que le réactif (3) est formé par des lymphocytes de culture portant de la ferritine.

16. Méthode selon la revendication 2, caractérisée en ce qu'en plus des opération (a) à (h) précitées est effectuée l'opération (i) suivante:

(i) répétition des opérations (a) à (h), mais en substituant des globules sanguins mononucléaires périphériques portant une substance protéinique connue pour produire une réaction négative avec l'anticorps oncofoetal pour les globules sanguins mononucléaires périphériques portant de l'antigène oncofoetal.